# EUROPEAN PATENT APPLICATION

(11) **EP 4 821 934 A1**
(43) Date of publication of application: **14.10.2026**
(21) Application number: 24900106.6
(22) Date of filing: 05.12.2024
(51) Int. Cl.: C07D 495/04, C07D 453/04, A61K 31/4436

(54) **INTERMEDIATE COMPOUND USED IN PREPARING HETEROCYCLE DERIVATIVE, METHOD FOR PREPARING INTERMEDIATE COMPOUND, AND METHOD FOR PREPARING HETEROCYCLE DERIVATIVE USING SAME**

(30) Priority: 08.12.2023 KR 20230177323
(71) Applicant: JW Pharmaceutical Corporation, Gyeonggi-do 13840 (KR)
(72) Inventor: YOON, Dooha, Seongnam-si, Gyeonggi-do 13562 (KR); PARK, Suha, Seoul 05224 (KR); LEE, Youngju, Seoul 01863 (KR); CHIN, Seiho, Hwaseong-si, Gyeonggi-do 18307 (KR)
(74) Representative: HGF
(86) International application number: PCT/IB2024/062239
(87) International publication number: WO 2025/120550

(57) **Abstract**

The present invention relates to: an intermediate compound used in preparing a heterocycle derivative; a method for preparing same; and a method for preparing a heterocycle derivative using same.

## Description

### Technical Field

The present invention relates to an intermediate compound used for preparing a heterocycle derivative, a method for preparing the same, and a method for preparing the heterocycle derivative using the same.

### Background

Signal transducer and activator of transcription (STAT) proteins are transcription factors which transmit signals from various extracellular cytokines and growth factors into nucleuses, and a total of seven subtypes (STAT1, STAT2, STAT3, STAT4, STAT5a, STAT5b, STAT6) have been reported so far.

In particular, the STAT3 proteins are known to play a key role in the IL-6 and EGF signaling systems. It has also been reported that the STAT3 proteins are continuously activated in patients with solid cancer, which occurs in the prostate, stomach, breast, lung, pancreas, kidney, uterus, ovary, head and neck, etc., and patients with blood cancer such as acute and chronic leukemia, multiple myeloma, etc. In many studies, STAT3 is considered a highly promising novel anticancer target, since apoptosis induced by anti-sensing STAT3 has been observed in a variety of cancer cell lines.

In addition, the present inventors have recently confirmed that the compound of formula I below has an excellent effect of inhibiting activity against the STAT3 proteins, and thus may be advantageously used for the treatment of cancer.

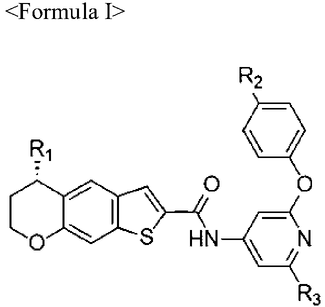

The compound of formula I is a drug having optical activity, and thus essentially requires a process of optically separating a specific form during a preparation process. In particular, there is a need for a preparation method with stably excellent yield and optical purity while reducing costs, the number of processes, process time, and the like, in mass production.

### Related Art References

### Patent Documents

(Patent Document 1) Korean Publication of Patent Application No. 10-2017-0081708

### Detailed Description of the Invention

### Technical Problem

The present invention provides a method for preparing a compound of formula I or a solvate thereof with stably excellent yield and optical purity.

The present invention provides an intermediate compound used for preparing a compound of formula I or a solvate thereof, and a method for preparing the same.

### Technical Solution

The present invention provides a method for preparing a compound of formula I or a solvate thereof, the method including the following steps:
a step of preparing a compound represented by formula 2 using a compound represented by formula 1;
a step of preparing a compound represented by formula 1a using the compound represented by formula 2; and
a step of preparing a compound represented by formula I or a solvate thereof using the compound represented by formula 1a and a compound represented by formula 3: 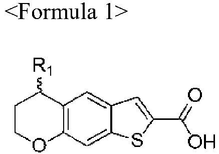 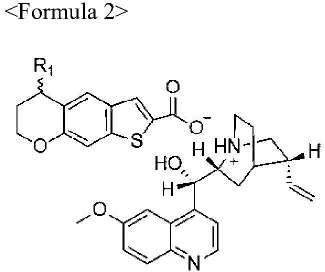 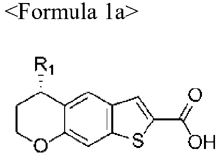 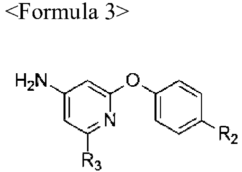 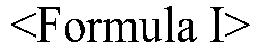 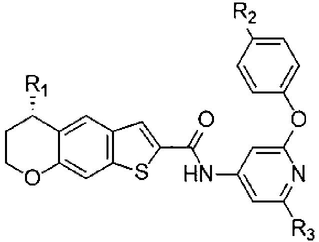
wherein in formulas 1, 2, 1a, 3 and I,
R₁ is -S(=O)(=Ra)Rb,
Ra is O or NH,
Rb is H, C1-6 alkyl, C1-6 alkoxy-C 1-6 alkyl, C1-6 alkylcarbonyl-C1-6 alkyl, C2-7 alkenyl, amino, or amino C1-6 alkyl,
at least one H of Ra or Rb is each independently substituted with F, Br, Cl or I, and
R₂ and R₃ are each independently F, Br, Cl or I.

In the present invention, the compound represented by formula 1 means a mixture including the compound represented by formula 1a and the compound represented by formula 1b.

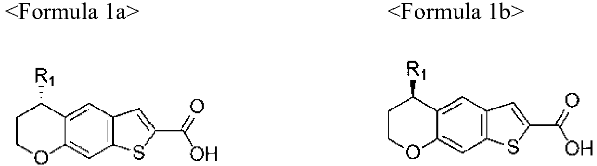

In the present invention, the compound represented by formula 2 means a mixture including the compound represented by formula 2a and the compound represented by formula 2b.

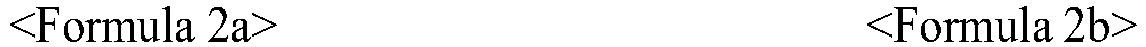

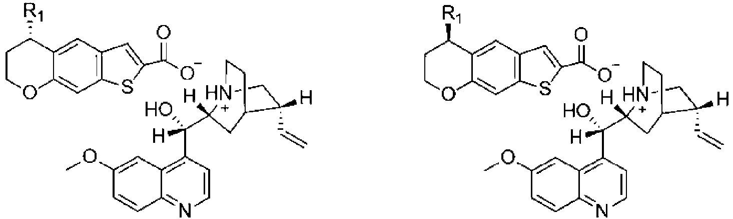

In one example, the step of preparing of the compound represented by formula 2 using the compound represented by formula 1 may include a step of reacting the compound represented by formula 1 with quinidine.

In the present invention, quinidine means a compound having the IUPAC name of (S)-(6-methoxyquinolin-4-yl)((1S,2R,4S,5R)-5-vinylquinuclidin-2-yl)methanol.

In one example, the compound represented by formula 1 and quinidine may be reacted in the presence of methanol (MeOH), ethanol (ETOH), propanol (PrOH), isopropanol (i-PrOH), butanol (BuOH), or a mixture thereof, and most preferably methanol or ethanol.

In one example, the step of preparing of the compound represented by formula 2 using the compound represented by formula 1 may include reacting the compound represented by formula 1 with quinidine at 0 to 90°C.

In one example, the compound represented by formula 1 and quinidine may be mixed at an equivalent ratio of 1:0.8 to 1:1.3. For example, the compound represented by formula 1 and quinidine may be mixed at an equivalent ratio of 1:0.85 to 1:1.25. For example, the compound represented by formula 1 and quinidine may be mixed at an equivalent ratio of 1:0.9 to 1:1.2. For example, the compound represented by formula 1 and quinidine may be mixed at an equivalent ratio of 1:1 to 1:1.1. For example, the compound represented by formula 1 and quinidine may be mixed at an equivalent ratio of 1:1.05.

In one example, in the step of preparing of the compound represented by formula 2 by reacting the compound represented by formula 1 with quinidine in the presence of methanol, ethanol, propanol, isopropanol, butanol, or a mixture thereof, above methanol, ethanol, propanol, isopropanol, butanol, or the mixture thereof may be mixed in a volume of 1 to 100 times the weight of the compound represented by formula 1. For example, methanol, ethanol, propanol, isopropanol, butanol, or a mixture thereof may be mixed in a volume of 1 to 80 times the weight of the compound represented by formula 1. For example, methanol, ethanol, propanol, isopropanol, butanol, or a mixture thereof may be mixed in a volume of 1 to 70 times the weight of the compound represented by formula 1. For example, methanol, ethanol, propanol, isopropanol, butanol, or a mixture thereof may be mixed in a volume of 1 to 60 times the weight of the compound represented by formula 1.

In one example, in the step of preparing of the compound represented by formula 2 by reacting the compound represented by formula 1 with quinidine in the presence of methanol, above methanol may be mixed in a volume of 1 to 40 times the weight of the compound represented by formula 1. For example, methanol may be mixed in a volume of 2 to 35 times the weight of the compound represented by formula 1. For example, methanol may be mixed in a volume of 3 to 30 times the weight of the compound represented by formula 1. For example, methanol may be mixed in a volume of 4 to 30 times the weight of the compound represented by formula 1. For example, methanol may be mixed in a volume of 5 to 30 times the weight of the compound represented by formula 1.

In one example, in the step of preparing of the compound represented by formula 2 by reacting the compound represented by formula 1 with quinidine in the presence of ethanol, above ethanol may be mixed in a volume of 3 to 60 times the weight of the compound represented by formula 1. For example, ethanol may be mixed in a volume of 5 to 55 times the weight of the compound represented by formula 1. For example, ethanol may be mixed in a volume of 10 to 50 times the weight of the compound represented by formula 1. For example, ethanol may be mixed in a volume of 15 to 50 times the weight of the compound represented by formula 1. For example, ethanol may be mixed in a volume of 20 to 50 times the weight of the compound represented by formula 1.

In one example, the compound represented by formula 1 may be a compound represented by formula 1-1, and the compound represented by formula 2 may be a compound represented by formula 2-1.

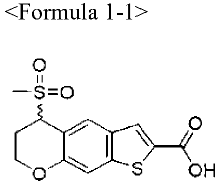

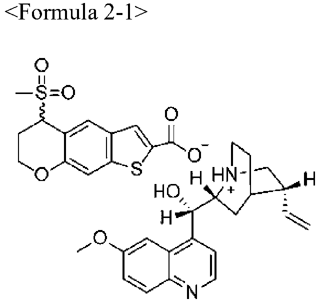

In one example, the step of preparing of the compound represented by formula 2-1 by reacting the compound represented by formula 1-1 with quinidine in the presence of methanol or ethanol may be represented by reaction formula 1 below.

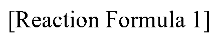

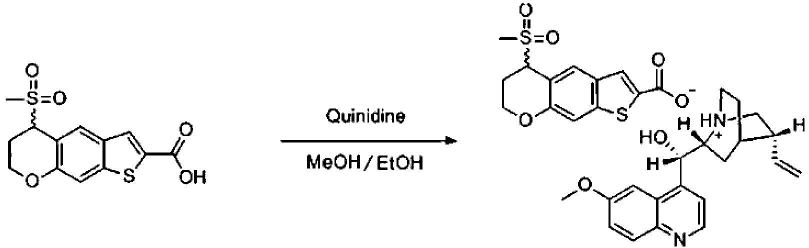

In one embodiment form, the step of preparing of the compound represented by formula 2-1 by reacting the compound represented by formula 1-1 with quinidine in the presence of methanol may be performed by mixing the compound represented by formula 1-1 and quinidine in an equivalent ratio of 1:1.1, and mixing methanol in a volume of 5 times the weight of the compound represented by formula 1-1.

In one example, a step of recovering quinidine may be further included after the step of preparing of the compound represented by formula 2 using the compound represented by formula 1.

In one example, a step of recovering the compound represented by formula 1 may be further included after the step of preparing of the compound represented by formula 2 using the compound represented by formula 1.

In one example, a step of recovering quinidine and a step of the compound represented by formula 1 may be further included after the step of preparing of the compound represented by formula 2 using the compound represented by formula 1.

In one example, a step of recovering quinidine may be further included between the step of preparing of the compound represented by formula 2 using the compound represented by formula 1 and the step of preparing of the compound represented by formula 1a using the compound represented by formula 2.

In one example, a step of recovering the compound represented by formula 1 may be further included between the step of preparing of the compound represented by formula 2 using the compound represented by formula 1 and the step of preparing of the compound represented by formula 1a using the compound represented by formula 2.

In one example, a step of recovering quinidine and a step of the compound represented by formula 1 may be further included between the step of preparing of the compound represented by formula 2 using the compound represented by formula 1 and the step of preparing of the compound represented by formula 1a using the compound represented by formula 2.

In one example, it may include: a step of preparing a compound represented by formula 2 using a compound represented by formula 1;
a step of recovering quinidine;
a step of recovering the compound represented by formula 1; and
a step of preparing a compound represented by formula 1a using the compound represented by formula 2.

In one example, it may include: a step of preparing a compound represented by formula 2 using a compound represented by formula 1;
a step of recovering quinidine;
a step of recovering the compound represented by formula 1;
a step of preparing a compound represented by formula 2 using the compound represented by formula 1; and
a step of preparing a compound represented by formula 1a using the compound represented by formula 2.

In one example, the step of preparing of the compound represented by formula 2 using the compound represented by formula 1; the step of recovering of quinidine; and the step of recovering of the compound represented by formula 1 may be repeatedly performed.

In one example, after the step of preparing of the compound represented by formula 2 using the compound represented by formula 1; the step of recovering of quinidine; and the step of recovering of the compound represented by formula 1 are repeatedly performed, the step of preparing of the compound represented by formula 2 using the compound represented by formula 1, and the step of preparing of the compound represented by formula 1a using the compound represented by formula 2 may be performed.

In one example, the step of preparing of the compound represented by formula 2 using the compound represented by formula 1; the step of recovering of quinidine; and the step of recovering of the compound represented by formula 1 may be performed repeatedly twice or more. For example, they may be performed repeatedly three times or more. For example, they may be performed repeatedly four times or more. For example, they may be performed repeatedly five times or more. For example, they may be performed repeatedly six times or more.

In one example, the step of preparing of the compound represented by formula 2 using the compound represented by formula 1; the step of recovering of quinidine; and the step of recovering of the compound represented by formula 1 may be performed repeatedly ten times or less. For example, they may be performed repeatedly nine times or less. For example, they may be performed repeatedly eight times or less. For example, they may be performed repeatedly seven times or less.

In one example, the step of preparing of the compound represented by formula 2 using the compound represented by formula 1; the step of recovering of quinidine; and the step of recovering of the compound represented by formula 1 may be performed repeatedly once or more and ten times or less. For example, they may be performed repeatedly once or more and eight times or less. For example, they may be performed repeatedly once or more and six times or less. For example, they may be performed repeatedly once or more and five times or less. For example, they may be performed repeatedly twice or more and ten times or less. For example, they may be performed repeatedly twice or more and eight times or less. For example, they may be performed repeatedly twice or more and six times or less. For example, they may be performed repeatedly twice or more and five times or less.

In one embodiment, the step of recovering of quinidine may be performed by adding 1,8-diazabicyclo[5.4.0]undec-7-ene (DBU).

In one example, the step of recovering of quinidine may be performed in the presence of methanol, ethanol, propanol, isopropanol, or a mixture thereof, and most preferably methanol.

In one example, the step of recovering of quinidine may be performed by adding the DBU in the presence of methanol, ethanol, propanol, isopropanol, or a mixture thereof.

In one example, the step of recovering of quinidine may be performed by adding the DBU to the compound represented by formula 2b in the presence of methanol, ethanol, propanol, isopropanol, or a mixture thereof.

In one example, the step of recovering of quinidine may be performed to obtain the compound represented by formula 1 and quinidine.

In one example, the step of recovering of quinidine may be performed by adding the DBU to the compound represented by formula 2b in the presence of methanol, ethanol, propanol, isopropanol, or a mixture thereof, so as to obtain the compound represented by formula 1 and quinidine.

In one example, the step of recovering of the compound represented by formula 1 may be performed by adding an acid.

In one example, the step of recovering of the compound represented by formula 1 may be performed by adding the acid to the compound represented by formula 1 in a dissolved state.

In one example, the step of recovering of the compound represented by formula 1 may be performed by adding hydrochloric acid (HCl), sulfuric acid, acetic acid, or trifluoroacetic acid, and preferably HCl.

In one example, the step of recovering of the compound represented by formula 1 may be performed to obtain the compound, which is represented by formula 1 in the dissolved state, as a solid.

In one example, the step of recovering of the compound represented by formula 1 may be performed to obtain the compound represented by formula 1 in the solid state from the compound represented by formula 1 in the dissolved state.

In one example, the step of recovering of the compound represented by formula 1 may be performed by adding the acid to the compound represented by formula 1 in the dissolved state, so as to obtain the compound represented by formula 1 in the solid state.

In the method for preparing the compound of formula I or the solvate thereof according to the present invention, the compound of formula I or the solvate thereof may be prepared at a high yield by repeating the synthesis process and the recovery process as described above, and the compound of formula I or the solvate thereof may be economically prepared by reducing preparation costs.

In one example, the step of preparing of the compound represented by formula 1a using the compound represented by formula 2 may be a desalting process.

In one example, the step of preparing of the compound represented by formula 1a using the compound represented by formula 2 may be performed by adding the acid.

In one example, the step of preparing of the compound represented by formula 1a using the compound represented by formula 2 may be performed by adding hydrochloric acid, sulfuric acid, acetic acid, or trifluoroacetic acid, and preferably hydrochloric acid.

In one example, the step of preparing of the compound represented by formula 1a using the compound represented by formula 2 may be performed in the presence of methanol, ethanol, propanol, isopropanol, or a mixture thereof, and preferably methanol.

In one example, the step of preparing of the compound represented by formula 1a using the compound represented by formula 2 may be performed by adding hydrochloric acid, sulfuric acid, acetic acid, or trifluoroacetic acid in the presence of methanol, ethanol, propanol, isopropanol, or a mixture thereof.

In one example, the step of preparing of the compound represented by formula 1a using the compound represented by formula 2 may be performed by adding hydrochloric acid, sulfuric acid, acetic acid, or trifluoroacetic acid in the presence of methanol.

In one example, the step of preparing of the compound represented by formula 1a using the compound represented by formula 2 may be performed by adding hydrochloric acid in the presence of methanol, ethanol, propanol, isopropanol, or a mixture thereof.

In one example, in the step of preparing of the compound represented by formula 1a using the compound represented by formula 2, methanol, ethanol, propanol, isopropanol, or the mixture thereof may be mixed in a volume of 1 to 20 times the weight of the compound represented by formula 2. For example, methanol, ethanol, propanol, isopropanol, or the mixture thereof may be mixed in a volume of 1 to 15 times the weight of the compound represented by formula 2. For example, methanol, ethanol, propanol, isopropanol, or the mixture thereof may be mixed in a volume of 1 to 10 times the weight of the compound represented by formula 2.

In one example, in the step of preparing of the compound represented by formula 1a using the compound represented by formula 2, methanol may be mixed in a volume of 3 to 10 times the weight of the compound represented by formula 2. For example, methanol may be mixed in a volume of 4 to 8 times the weight of the compound represented by formula 2. For example, methanol may be mixed in a volume of 4 to 6 times the weight of the compound represented by formula 2.

In one example, in the step of preparing of the compound represented by formula 1a using the compound represented by formula 2, hydrochloric acid, sulfuric acid, acetic acid, and trifluoroacetic acid may have a concentration of 1 N to 20 N. For example, hydrochloric acid, sulfuric acid, acetic acid, and trifluoroacetic acid may have a concentration of 1 N to 10 N. For example, hydrochloric acid, sulfuric acid, acetic acid, and trifluoroacetic acid may have a concentration of 1 N to 5 N.

In one example, in the step of preparing of the compound represented by formula 1a using the compound represented by formula 2, hydrochloric acid may have a concentration of 1 N to 5 N. For example, hydrochloric acid may have a concentration of 1 N to 4 N. For example, hydrochloric acid may have a concentration of 1 N to 3 N. For example, hydrochloric acid may have a concentration of 1 N to 2 N.

In one example, in the step of preparing of the compound represented by formula 1a using the compound represented by formula 2, hydrochloric acid, sulfuric acid, acetic acid, or trifluoroacetic acid may be mixed in a volume of 1 to 100 times the weight of the compound represented by formula 2. For example, hydrochloric acid, sulfuric acid, acetic acid, or trifluoroacetic acid may be mixed in a volume of 1 to 80 times the weight of the compound represented by formula 2. For example, hydrochloric acid, sulfuric acid, acetic acid, or trifluoroacetic acid may be mixed in a volume of 1 to 70 times the weight of the compound represented by formula 2. For example, hydrochloric acid, sulfuric acid, acetic acid, or trifluoroacetic acid may be mixed in a volume of 1 to 60 times the weight of the compound represented by formula 2.

In one example, in the step of preparing of the compound represented by formula 1a using the compound represented by formula 2, hydrochloric acid may be mixed in a volume of 1 to 60 times the weight of the compound represented by formula 2. For example, hydrochloric acid may be mixed in a volume of 3 to 55 times the weight of the compound represented by formula 2. For example, hydrochloric acid may be mixed in a volume of 5 to 50 times the weight of the compound represented by formula 2. For example, hydrochloric acid may be mixed in a volume of 10 to 50 times the weight of the compound represented by formula 2.

In one example, in the step of preparing of the compound represented by formula 1a using the compound represented by formula 2, hydrochloric acid having a concentration of 1 N to 2 N may be mixed in a volume of 5 to 50 times the weight of the compound represented by formula 2. Preferably, hydrochloric acid having a concentration of 2 N may be mixed in a volume of 10 to 50 times the weight of the compound represented by formula 2.

In one example, the step of preparing of the compound represented by formula 1a using the compound represented by formula 2 may be performed at 0 to 40°C.

In one example, the compound represented by formula 2 may be a compound represented by formula 2-1, and the compound represented by formula 1a may be a compound represented by formula 1a-1.

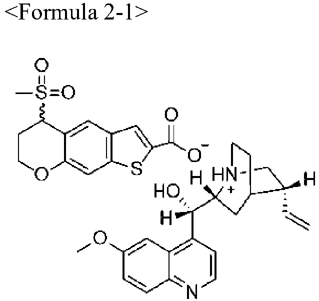

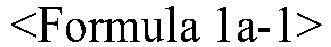

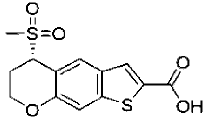

In one example, a step of preparing of the compound represented by formula 1a-1 using the compound represented by formula 2-1 may be represented by reaction formula 2 below.

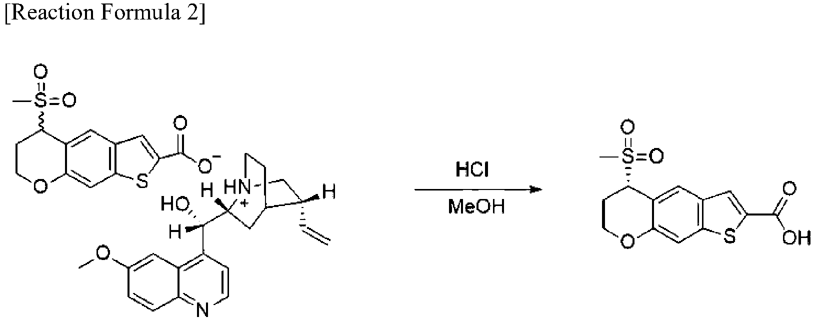

In one embodiment form, the step of preparing of the compound represented by formula 1a-1 using the compound represented by formula 2-1 may include a step of obtaining a compound represented by formula 2a-1 using the compound represented by formula 2-1 and a step of preparing the compound represented by formula 1a-1 through a desalting process of the compound. In this case, the step of obtaining of the compound represented by formula 2a-1 using the compound represented by formula 2-1 may be performed by washing with the compound represented by formula 2-1 and methanol in a volume of twice the weight of said compound, followed by filtration. In addition, the step of preparing of the compound represented by formula 1a-1 through a desalting process of the compound represented by formula 2a-1 may be performed by reacting the compound represented by formula 2a-1, and methanol in a volume of five times and hydrochloric acid having a concentration of 2 N in a volume of 50 times based on the weight of said compound at 20 to 30°C, followed by filtration and drying under vacuum.

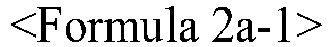

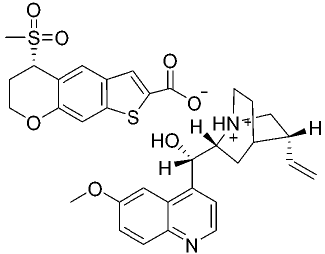

In one example, the step of preparing of the compound represented by formula I or the solvate thereof using the compound represented by formula 1a and the compound represented by formula 3 may be performed through an amidation reaction.

In one example, the amidation reaction may be performed through various methods known in the art.

In one example, the step of preparing of the compound represented by formula I or the solvate thereof using the compound represented by formula 1a and the compound represented by formula 3 may be performed by adding phosphorus oxychloride (POCl₃) in the presence of pyridine. In this case, purity may be improved.

In one example, the step of preparing of the compound represented by formula I or the solvate thereof using the compound represented by formula 1a and the compound represented by formula 3 may be performed at -10 to 30°C. Preferably, it may be performed at -10 to 20°C.

In one example, the compound represented by formula 1a may be a compound represented by formula 1a-1, the compound represented by formula 3 may be a compound represented by formula 3-1, and the compound represented by formula I may be a compound represented by formula I-1.

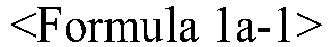

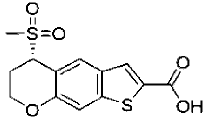

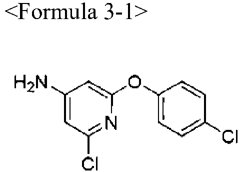

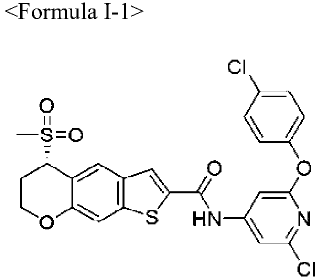

In one example, the step of preparing of the compound represented by formula I-1 or the solvate thereof using the compound represented by formula 1a-1 and the compound represented by formula 3-1 may be represented by reaction formula 3 below.

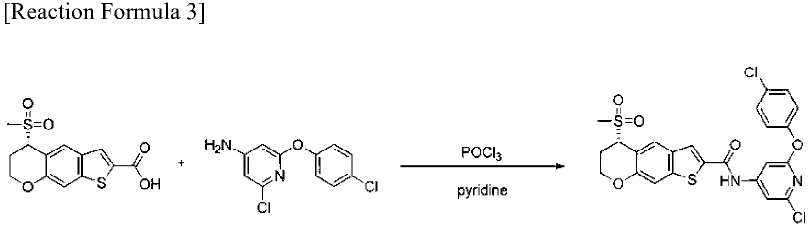

In one embodiment form, the step of preparing of the compound represented by formula I-1 using the compound represented by formula 1a-1 and the compound represented by formula 3-1 may be performed by mixing the compound represented by formula 1a-1 and the compound represented by formula 3-1 in an equivalent ratio of 1:1.2, adding thereto pyridine in a volume of 10 times and water in a volume of 0.06 times based on the weight of the compound represented by formula 1a-1, followed by cooling, and adding POCl₃ in three portions by 0.5 equivalents based on the weight of the compound represented by formula 1a-1.

In one example, the step of preparing of the compound represented by formula I or the solvate thereof using the compound represented by formula 1a and the compound represented by formula 3 may further include a step of preparing of the solvate of the compound represented by formula I using the compound represented by formula I.

In one example, the step of preparing of the solvate of the compound represented by formula I using the compound represented by formula I may be performed by adding tetrahydrofuran (THF) and water.

In one example, the compound represented by formula I may be a compound represented by formula I-1.

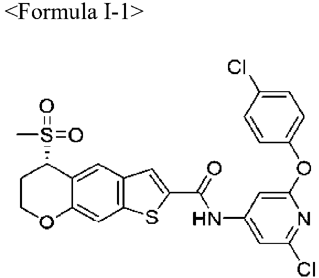

In one example, the step of preparing of a salt of the compound represented by formula I-1 using the compound represented by formula I-1 may be represented by reaction formula 4 below.

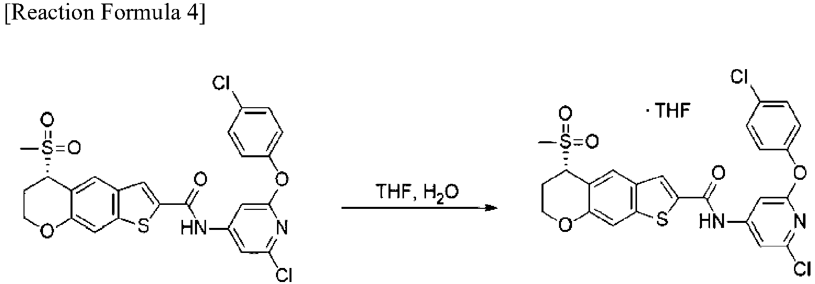

In one example, the compound represented by formula 1 may be the compound represented by formula 1-1, the compound represented by formula 2 may be the compound represented by formula 2-1, the compound represented by formula 1a may be the compound represented by formula 1a-1, the compound represented by formula 3 may be the compound represented by formula 3-1, and the compound represented by formula I may be the compound represented by Formula I-1.

In one example, the method for preparing the compound represented by formula I-1 or the solvate thereof according to the present invention may be performed according to reaction formula 5 below.

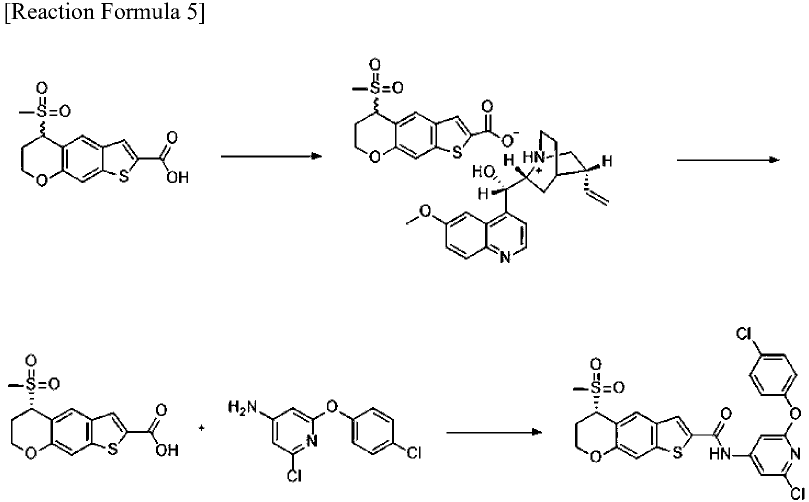

In one example, the compound represented by formula 1-1 may be prepared through a method including the following steps:
a step of preparing a compound represented by formula 6 using a compound represented by formula 4 and a compound represented by formula 5;
a step of preparing a compound represented by formula 7 using the compound represented by formula 6;
a step of preparing a compound represented by formula 8 using the compound represented by formula 7; and
a step of preparing a compound represented by formula 1-1 using the compound represented by formula 8.

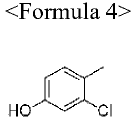

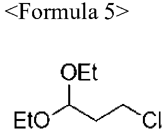

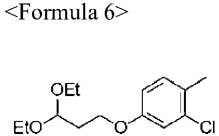

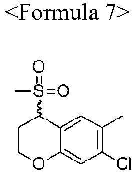

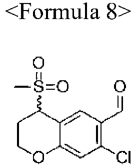

In one example, the step of preparing of the compound represented by formula 6 using the compound represented by formula 4 and the compound represented by formula 5 may be performed by treating the compound represented by formula 4 and the compound represented by formula 5 with a base in the presence of an organic solvent.

In one example, the organic solvent may be dimethylformamide (DMF), dimethylacetamide (DMAc), tetrahydrofuran, 2-methyltetrahydrofuran, acetonitrile (ACN), toluene (Tol), or a mixture thereof, and most preferably the DMF.

In one example, the base may be potassium hydroxide (KOH), potassium carbonate (K₂CO₃), or cesium carbonate (Cs₂CO₃).

In one example, the step of preparing of the compound represented by formula 6 using the compound represented by formula 4 and the compound represented by formula 5 may be represented by reaction formula 6 below.

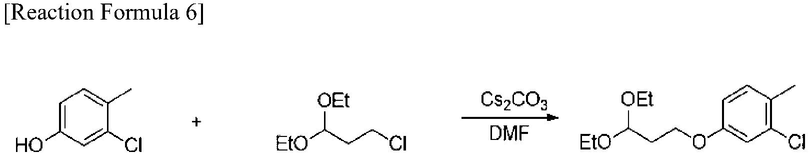

In one example, the step of preparing of the compound represented by formula 7 using the compound represented by formula 6 may be performed by adding sodium methansulfinate (MeSO₂Na) in the presence of trifluoroacetic acid (TFA) and dichloromethane (DCM).

In one example, the step of preparing of the compound represented by formula 7 using the compound represented by formula 6 may be performed by adding the compound represented by formula 6 to a mixed solution of trifluoroacetic acid and dichloromethane. In this case, production efficiency may be improved.

In one example, trifluoroacetic acid and dichloromethane may be mixed at a volume ratio of 1:1 to 5:1. For example, trifluoroacetic acid and dichloromethane may be mixed at a volume ratio of 1:1 to 4:1. For example, trifluoroacetic acid and dichloromethane may be mixed at a volume ratio of 1:1 to 3:1. For example, trifluoroacetic acid and dichloromethane may be mixed at a volume ratio of 2:1.

In one example, the step of preparing of the compound represented by formula 7 using the compound represented by formula 6 may be represented by reaction formula 7 below.

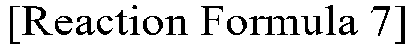

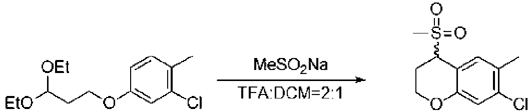

In one example, the step of preparing of the compound represented by formula 7 using the compound represented by formula 6 may include crystallizing with methanol. In this case, yield, quality and production efficiency may be improved.

In one example, the step of preparing of the compound represented by formula 8 using the compound represented by formula 7 may be performed by adding ceric ammonium nitrate (CAN) in the presence of a solvent.

In one example, in the step of preparing of the compound represented by formula 8 using the compound represented by formula 7, the solvent may be water, methanol, ethanol, propanol, isopropanol, butanol, acetonitrile, tetrahydrofuran, 1,4-dioxane, or a mixture thereof, and preferably ethanol.

In one example, in the step of preparing of the compound represented by formula 8 using the compound represented by formula 7, a large amount of the compound represented by formula 8 may be prepared at an excellent yield through a relatively simple process by adding ceric ammonium nitrate (CAN) in the presence of ethanol.

In one example, the step of preparing of the compound represented by formula 8 using the compound represented by formula 7 may be represented by reaction formula 8 below.

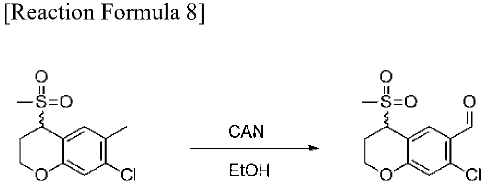

In one example, the step of preparing of the compound represented by formula 1-1 using the compound represented by formula 8 may be performed by adding methyl thioglycolate and cesium carbonate (Cs₂CO₃) in the presence of dimethylformamide.

In one example, methyl thioglycolate may be added in portions. For example, 1.5 equivalents of methyl thioglycolate may be slowly added dropwise thereto, the reaction solution may be warmed, 0.5 equivalents of methyl thioglycolate may be further added thereto after one hour, and 0.5 equivalents of methyl thioglycolate may be added thereto once more at intervals of 30 minutes based on 1 equivalent of the compound represented by formula 8. In this case, yield may be improved.

In one example, the step of preparing of the compound represented by formula 1-1 using the compound represented by formula 8 may include crystallizing at pH 2 to 3 and at 15 to 25°C. In this case, yield may be improved.

In one example, the step of preparing of the compound represented by formula 1-1 using the compound represented by formula 8 may be represented by reaction formula 9 below.

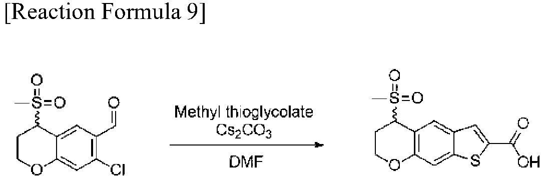

In one example, the method for preparing the compound represented by formula 1-1 according to the present invention may be performed according to reaction formula 10 below.

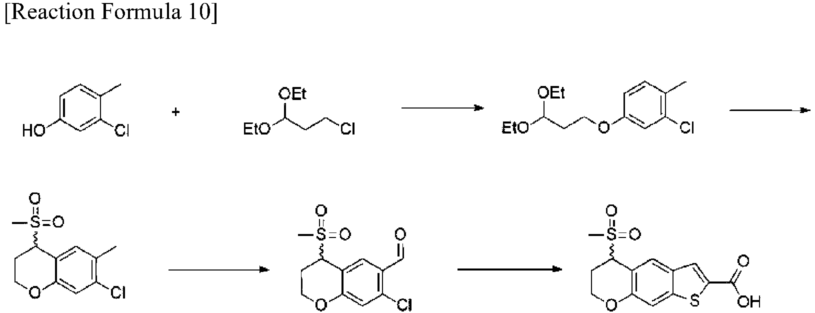

The present invention provides an intermediate compound represented by formula 2 below for preparing a compound represented by formula I or a solvate thereof: 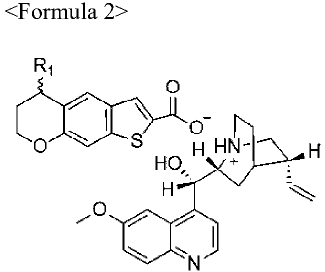 wherein in formula 2,
R₁ is -S(=O)(=Ra)Rb,
Ra is O or NH,
Rb is H, C1-6 alkyl, C1-6 alkoxy-C 1-6 alkyl, C1-6 alkylcarbonyl-C1-6 alkyl, C2-7 alkenyl, amino, or amino C1-6 alkyl, and
at least one H of Ra or Rb is each independently substituted with F, Br, Cl or I.

The present invention provides a use of the intermediate compound represented by above formula 2 for preparing the compound represented by formula I or the solvate thereof.

In one example, the intermediate compound represented by formula 2 may be a compound represented by formula 2-1.

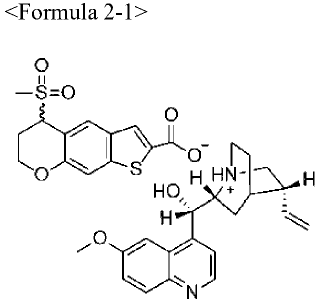

In one example, an intermediate compound represented by formula 2-1 may be used for preparing a compound represented by formula I-1 or a solvate thereof.

In the present invention, a compound represented by formula 2 may be a stable material, and may be easily obtained as a solid without a separate purification process. The compound represented by formula 2 may have excellent stability and thus may be stably and easily used in the preparation of the compound of formula I or the solvate thereof. In addition, the compound represented by formula 2 according to the present invention may have a good chiral resolution effect. In other words, a compound represented by formula 1 may be easily subjected to chiral resolution.

The present invention provides a method for preparing an intermediate compound for preparing a compound represented by formula I or a solvate thereof, the method including a step of preparing a compound represented by formula 2 using a compound represented by formula 1: 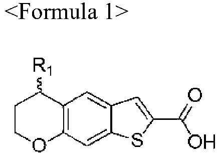 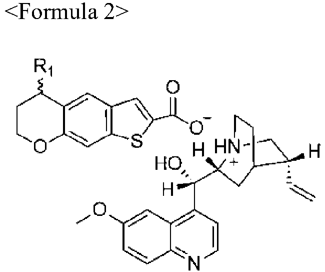 wherein in formulas 1 and 2,
R₁ is -S(=O)(=Ra)Rb,
Ra is O or NH,
Rb is H, C1-6 alkyl, C1-6 alkoxy-C 1-6 alkyl, C1-6 alkylcarbonyl-C1-6 alkyl, C2-7 alkenyl, amino, or amino C1-6 alkyl, and
at least one H of Ra or Rb is each independently substituted with F, Br, Cl or I.

In one example, the compound represented by formula 1 may be a compound represented by formula 1-1, and the compound represented by formula 2 may be a compound represented by formula 2-1.

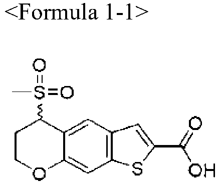

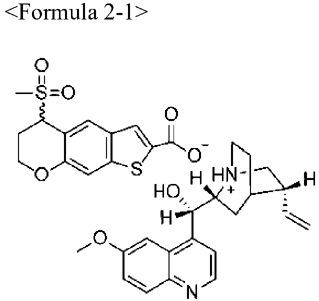

The method for preparing the compound represented by above formula 2 is substantially the same as described in the preparing of the compound represented by formula 2 in the method for preparing the compound of formula I or the solvate thereof. Thus, any redundant detailed description will be omitted. For example, the compound represented by above formula 2-1 may be prepared according to above reaction formula 1.
(1) The present invention provides a method for preparing a compound represented by formula I or a solvate thereof, the method including the following steps:
   a step of preparing a compound represented by formula 2 using a compound represented by formula 1;
   a step of preparing a compound represented by formula 1a using the compound represented by formula 2; and
   a step of preparing a compound represented by formula I or a solvate thereof using the compound represented by formula 1a and a compound represented by formula 3: 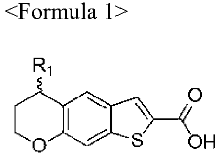 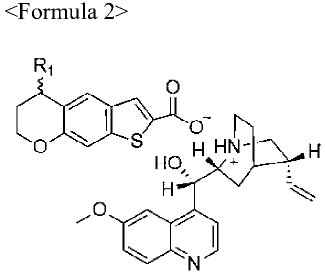 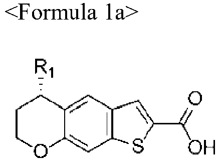 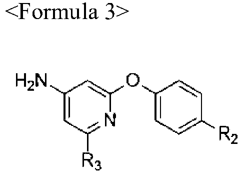 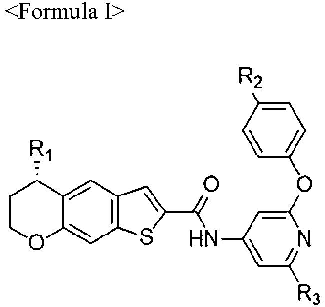
   wherein in formulas 1, 2, 1a, 3 and I,
   R₁ is -S(=O)(=Ra)Rb,
   Ra is O or NH,
   Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
   at least one H of Ra or Rb is each independently substituted with F, Br, Cl or I, and
   R₂ and R₃ are each independently F, Br, Cl or I.
(2) In (1), the step of preparing of the compound represented by formula 2 using the compound represented by formula 1 may include reacting the compound represented by formula 1 with quinidine.
(3) In (1) or (2), the step of preparing of the compound represented by formula 2 using the compound represented by formula 1 may include reacting the compound represented by formula 1 with quinidine in the presence of methanol, ethanol, propanol, isopropanol, butanol, or a mixture thereof.
(4) In any one of (1) to (3), the step of preparing of the compound represented by formula 2 using the compound represented by formula 1 may include reacting the compound represented by formula 1 with quinidine at 0 to 90°C.
(5) In any one of (2) to (4), the compound represented by formula 1 and quinidine may be mixed at an equivalent ratio of 1:0.8 to 1:1.3.
(6) In any one of (3) to (5), methanol may be mixed in a volume of 1 to 40 times the weight of the compound represented by formula 1.
(7) In any one of (3) to (6), ethanol may be mixed in a volume of 3 to 60 times the weight of the compound represented by formula 1.
(8) In any one of (1) to (7), a step of recovering quinidine and a step of recovering the compound represented by formula 1 may be further included between the step of preparing of the compound represented by formula 2 using the compound represented by formula 1 and the step of preparing of the compound represented by formula 1a using the compound represented by formula 2.
(9) In (8), the step of preparing of the compound represented by formula 2 using the compound represented by formula 1, the step of recovering of quinidine, and the step of recovering of the compound represented by formula 1 may be repeated.
(10) In (9), the repeating may be performed twice or more and ten times or less.
(11) In any one of (8) to (10), the step of recovering of quinidine may be performed by adding 1,8-diazabicyclo[5.4.0]undec-7-ene.
(12) In any one of (8) to (11), the step of recovering of the compound represented by formula 1 may be performed by adding hydrochloric acid, sulfuric acid, acetic acid, or trifluoroacetic acid.
(13) In any one of (1) to (12), the step of preparing of the compound represented by formula 1a using the compound represented by formula 2 may be performed by adding hydrochloric acid, sulfuric acid, acetic acid, or trifluoroacetic acid.
(14) In any one of (1) to (13), the step of preparing of the compound represented by formula I or the solvate thereof using the compound represented by formula 1a and the compound represented by formula 3 may be performed through an amidation reaction.
(15) In any one of (1) to (14), the compound represented by formula 1 may be a compound represented by formula 1-1, the compound represented by formula 2 may be a compound represented by formula 2-1, the compound represented by formula 1a may be a compound represented by formula 1a-1, the compound represented by formula 3 may be a compound represented by formula 3-1, and the compound represented by formula I may be a compound represented by Formula I-1: 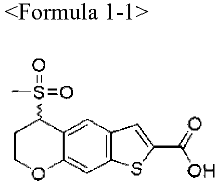 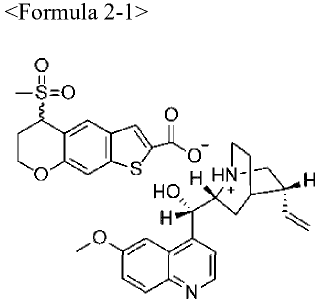 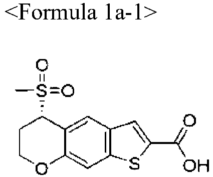 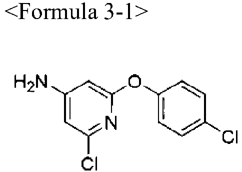 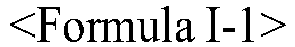 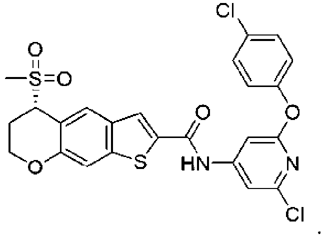
(16) In (15), the compound represented by formula 1-1 may be prepared by a method including:
   a step of preparing a compound represented by formula 6 using a compound represented by formula 4 and a compound represented by formula 5;
   a step of preparing a compound represented by formula 7 using the compound represented by formula 6;
   a step of preparing a compound represented by formula 8 using the compound represented by formula 7; and
   a step of preparing a compound represented by formula 1-1 using the compound represented by formula 8: 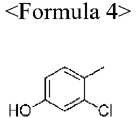 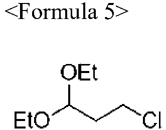 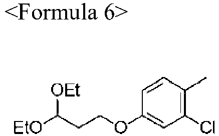 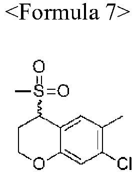 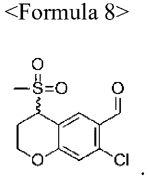
(17) In (15) or (16), the step of preparing of the compound represented by formula 1a-1 using the compound represented by formula 2-1 may include a step of obtaining a compound represented by formula 2a-1 using the compound represented by formula 2-1 and a step of preparing the compound represented by formula 1a-1 through a desalting process of the compound. In this case, the step of obtaining of the compound represented by formula 2a-1 using the compound represented by formula 2-1 may be performed by washing with the compound represented by formula 2-1 and methanol in a volume of twice the weight of said compound, followed by filtration. In addition, the step of preparing of the compound represented by formula 1a-1 through a desalting process of the compound represented by formula 2a-1 may be performed by reacting the compound represented by formula 2a-1, and methanol in a volume of five times and hydrochloric acid having a concentration of 2 N in a volume of 50 times based on the weight of said compound at 20 to 30°C, followed by filtration and drying under vacuum. 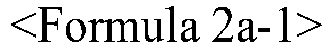 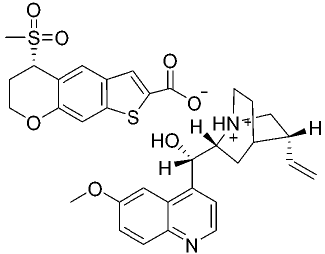
(18) The present invention provides an intermediate compound represented by formula 2 below for preparing a compound represented by formula I or a solvate thereof: 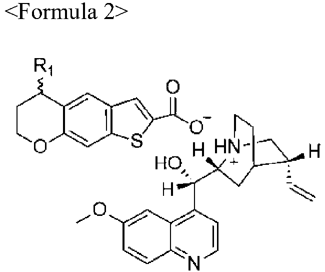 wherein in formula 2,
   R₁ is -S(=O)(=Ra)Rb,
   Ra is O or NH,
   Rb is H, C1-6 alkyl, C1-6 alkoxy-C 1-6 alkyl, C1-6 alkylcarbonyl-C1-6 alkyl, C2-7 alkenyl, amino, or amino C1-6 alkyl, and
   at least one H of Ra or Rb is each independently substituted with F, Br, Cl or I.
(19) In (18), a compound represented by formula 2 may be a compound represented by formula 2-1 below: 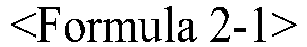 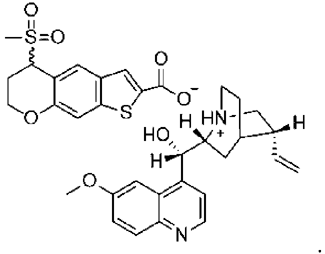
(20) The present invention provides a method for preparing an intermediate compound according to (17), the method including a step of preparing a compound represented by formula 2 using a compound represented by formula 1: 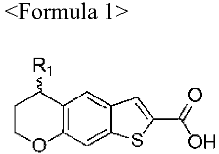 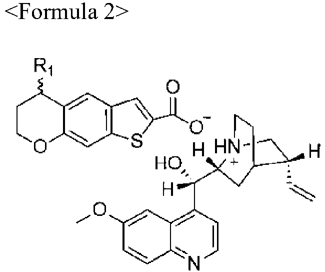 wherein in formulas 1 and 2,
   R₁ is -S(=O)(=Ra)Rb,
   Ra is O or NH,
   Rb is H, C1-6 alkyl, C1-6 alkoxy-C 1-6 alkyl, C1-6 alkylcarbonyl-C1-6 alkyl, C2-7 alkenyl, amino, or amino C1-6 alkyl, and
   at least one H of Ra or Rb is each independently substituted with F, Br, Cl or I.

### Advantageous Effects

An intermediate compound of the present invention can be a stable material, can be easily obtained as a solid without an additional purification process, can have excellent stability, and thus can be stably and easily used in the preparation of a compound of formula I or a solvate thereof. In addition, a compound represented by formula 1 can be easily subjected to chiral resolution therefrom.

According to a method for preparing the compound of formula I of the present invention or the solvate thereof, the compound of formula I or the solvate thereof can be prepared with high yield and high optical purity by a relatively simple process, and thus productivity can be improved. Furthermore, the present invention can be economical, convenient, and environmentally friendly since mass production is enabled, and costs such as preparation cost and time are reduced.

### Mode for Invention

Terms used in the present application are used only to describe a certain example and are not intended to limit the present invention. In the present application, terms such as "include," "have" or the like shall be intended to designate a presence of features, steps, operations, components, or combinations thereof described herein, and shall not be construed to exclude a possible presence or addition of one or more other features, steps, operations, components, or combinations thereof in advance. All the terms used herein including technical or scientific terms have the same meaning as commonly understood by those ordinary skilled in the art, to which the present invention pertains, unless defined otherwise.

The abbreviations used below are defined as follows.

| Abbreviation | Definition (full name) |
|---|---|
| DMF | Dimethylformamide |
| MTBE | Methyl tert-butyl ether |
| TFA | Trifluoroacetic acid |
| DCM | Dichloromethane |
| CAN | Ceric ammonium nitrate |
| DBU | 1,8-diazabicyclo[5.4.0]undec-7-ene |
| THF | Tetrahydrofuran |

### Preparation Example: Preparation of 2-chloro-6-(4-chlorophenoxy)pyridin-4-amine (formula 3-1)

4-amino-2,6-dichloropyridine (1.0 eq, 100 g), 4-chlorophenol (1.0 eq, 78.87 g), Cs₂CO₃ (1.0 eq, 199.89 g), and sulfolane (10 vol, 1000 ml) were added to a reactor, warmed to 165±5°C, and stirred for 8 to 12 hours. When 4-amino-2,6-dichloropyridine is < 15.0%, the reaction was completed, the inside of the reactor was cooled to 25±5°C, and then water (15 vol, 1500 ml) and MTBE (10 vol, 1000 ml) were added thereto to separate layers. MTBE (10 vol, 1000 ml) was added again to a separated aqueous layer to separate the layers, and sat. NaCl aqueous solution (5 vol, 500 ml) and water (5 vol, 500 ml) were added to an organic layer to separate layers. Na₂SO₄ (twice, 200 g) was added to the organic layer, followed by filtration and concentration, and then toluene (2 vol, 200 ml) was added again and completely concentrated. Toluene (3 vol, 300 ml) was added thereto, warmed to 80±5°C, stirred for 0.5 to 1 hour, cooled to 50±5°C, and stirred for four hours or more. The reaction solution was cooled to 25±5°C and stirred for two hours, after which the crystallized solution was filtered (washed with 50% toluene in heptane (2 vol, 200 ml)). If the title compound (4-amino-2,6-dichloropyridine < 0.2%, 2,6-bis(4-chlorophenoxy)pyridin-4-amine (dimer) imp. < 0.25%) was not suitable based on a solid state, the toluene purification process was repeated and dried under vacuum (yield 44.94%, purity 99.79%).

### Example: Preparing of compound of formula I or solvate thereof

### (Step 1) Synthesis of 2-chloro-4-(3,3-diethoxypropoxy)-1-methylbenzene (Formula 6)

3-chloro-4-methylphenol (formula 4) (1.0 eq, 100 g), Cs₂CO₃ (1.0 eq, 228.52 g), and 3-chloro-1,1-diethoxypropane (formula 5) (1.2 eq, 140.26 g) were added to DMF (10 vol, 1000 ml). The reaction solution was warmed to 120°C and stirred for three hours. When 3-chloro-4-methylphenol is < 1.0%, the reaction was completed, and MTBE (10 vol, 1000 ml) and water (15 vol, 1500 ml) were added to extract, and then an aqueous layer was re-extracted with MTBE (5 vol, 500 ml) again. The organic layer was washed with brine (15 vol, 1500 ml), dehydrated with Na₂SO₄ (twice, 200 g), filtered, concentrated, and dried to obtain the title compound (purity 93.68%, in-situ).

¹H NMR (500 MHz, DMSO-d₆)δppm 1.15(t, *J*=6.84Hz, 6H), 1.97-2.01 (m, 2H), 2.29 (s, 3H), 3.40-3.53 (m, 2H), 3.58-3.68 (m, 2H), 4.03(t, *J*=6.35Hz, 2H), 4.72(t, *J*=5.62Hz, 1H), 6.84-6.91(m, 1H), 7.01-7.06(m, 1H), 7.28 (d, *J*=8.30 Hz, 1H)

### (Step 2) Synthesis of 7-chloro-6-methyl-4-(methylsulfonyl)chromane (Formula 7)

TFA (3 vol, 600 ml) was added to DCM (8 vol, 1600 ml) and cooled to 0 to 10°C. The compound of formula 6 (1.0 eq, 200 g) obtained in above step 1 was dissolved in DCM (2 vol, 200 ml), slowly added dropwise to the DCM solution containing said TFA while maintaining the temperature, and stirred for 10 minutes. MeSO₂Na (1.5 eq, 112.27 g) was slowly added thereto while paying attention to heat generation, and then the resulting mixture was further stirred at 10 to 20°C for one hour. When the reaction was completed, water (8 vol, 1600 ml) was added to perform layer separation, and the aqueous layer was extracted once more with DCM (2 vol, 400 ml). The organic layer was collected again, washed with water (8 vol, 1600 ml), and saturated NaHCO₃ (5 vol, 1000 ml) was added to the organic layer to adjust to pH 8 to 9, and the organic layer was washed with water (8 vol, 1600 ml). Na₂SO₄ (twice, 400 g) was added thereto, dehydrated, and then filtered. At this time, DCM (2 vol, 400 ml) was used as a washing solution. The organic layer was all concentrated. Purification was performed by adding MeOH (10 vol, 2000 ml) and stirring for two to three hours under reflux, after which the resulting product was slowly cooled to 20 to 30°C again, stirred for 0.5 to 1 hour, and then filtered (washing solution MeOH (2 vol, 400 ml)) and dried under vacuum to obtain the title compound (total yield 50.47%, purity 98.08% in steps 1 and 2).

¹H NMR (500 MHz, DMSO-d₆)δppm 2.17-2.24(m, 1H), 2.25(s, 3H), 2.53-2.55(m, 1H), 3.12(s, 3H), 4.23 (dt, *J*=11.11, 3.24Hz, 1H), 4.30-4.40(m, 1H), 4.59(br d, *J*=4.89Hz, 1H), 6.97(s, 1H), 7.34(s, 1H)

### (Step 3) Synthesis of 7-chloro-4-(methylsulfonyl)chroman-6-carbaldehyde (Formula 8)

The compound of formula 7 (1.0 eq, 90 g) obtained in above step 2 and CAN (5.15 eq, 974.53 g) were added to EtOH (7 vol, 630 ml). The reaction solution was warmed to 40 to 50°C and stirred for four hours. When the reaction was completed (the compound of formula 7 obtained in above step 2 < 3%), the inside of the reactor was cooled to 25±5°C. Water (20 vol, 1800 ml) was added thereto, stirred at the same temperature for one hour, and then filtered and dried under vacuum to obtain the title compound (yield 86.79%, purity 96.66%).

¹H NMR (500 MHz, DMSO-d₆)δppm 2.23-2.33(m, 1H), 2.53-2.63(m, 1H), 3.17(s, 3H), 4.38-4.43(m, 1H), 4.45-4.51(m, 1H), 4.78(br d, *J*=4.89Hz, 1H), 7.18(s, 1H), 7.98(s, 1H), 10.19(s, 1H)

### (Step 4) Synthesis of 4-(methylsulfonyl)-3,4-dihydro-2H-thieno[3,2-g]chromen-7-carboxylic acid (Formula 1-1)

The compound of formula 8 (1 eq, 80 g) obtained in above step 3 and Cs₂CO₃ (3 eq, 284.64 g) were added to DMF (8 vol, 640 ml), and then methyl thioglycolate (1.5 eq, 46.6 g) was dissolved in DMF (2 vol, 160 ml) and slowly added dropwise to the reaction solution. The reaction solution was warmed to 70 to 80°C, methyl thioglycolate (0.5 eq, 15.45 g) was slowly added dropwise thereto in one hour after the temperature was raised, and methyl thioglycolate (0.5 eq, 15.45 g) was further added thereto once more at intervals of 30 minutes. When the reaction was completed, the reaction solution was cooled to 20 to 30°C, and water (20 vol, 1600 ml) and DCM (20 vol, 1600 ml) were added thereto and extracted. DCM (20 vol, 1600 ml) was added to the extracted aqueous layer and washed, and the reaction solution was cooled to 15 to 25°C and adjusted to pH 2 to 3 using 4 N HCl. The resulting mixture was stirred at the same temperature for one hour and filtered. The resulting product was washed once with EtOH (1 vol, 80 ml) and washed three times with hexane (3 vol, 240 ml), and the filtered solid was dried under vacuum to obtain the title compound (yield 92.95%, purity 96.70%).

¹H NMR (500 MHz, DMSO-d₆)δppm 2.27-2.36(m, 1H), 2.58-2.64(m, 1H), 3.16(s, 3H), 4.28-4.34(m, 1H), 4.43-4.50(m, 1H), 4.81(br d, *J*=3.91Hz, 1H), 7.53(s, 1H), 8.05(d, *J*=5.37Hz, 2H), 13.36(br s, 1H)

### (Step 5) Synthesis of (1S,2R,4S,5R)-2-((S)-hydroxy(6-methoxyquinolin-4-yl)methyl)-5-vinylquinuclidin-1-ium 4-(methylsulfonyl)-3,4-dihydro-2H-thieno[3,2-g]chromen-7-carboxylate (Formula 2-1)

(a) The compound of formula 1-1 (1.0 eq, 80 g) obtained in above step 4 and quinidine (1.1 eq, 91.40 g) and MeOH (5 vol, 400 ml) were added to a reactor. The reaction solution was warmed to 60±5°C and stirred for 30 minutes, then cooled to 25±5°C and stirred for three hours to obtain the title compound. After that, the resulting product was filtered (washed with cold MeOH (2 vol, 160 ml)) and dried under vacuum obtain the compound of formula 2a-1.
(b) DBU (1 eq, 39 g) was added to the filtrate, and the inside of the reactor was warmed to 50 to 55°C and stirred for 0.5 hours. IPC (chiral HPLC) was confirmed to complete the reaction when ee is < 2.0%. The reaction solution was cooled to 20±5°C and stirred for 0.5 to 1.0 hours, after which the precipitated solid was filtered and dried under vacuum to recover quinidine.
(c) The inside of the reactor containing the filtrate was cooled to 10°C or less. 1 N HCl (5 vol, 400 ml) was added thereto and adjusted to pH 1 to 2, and the resulting mixture was stirred for 0.5 to 1 hours while maintaining the inside of the reactor at 0 to 10°C. The precipitated solid was filtered, washed with MeOH (2 vol), and dried under vacuum to recover the compound of formula 1-1 obtained in above step 4.

The processes of (a) to (c) were repeated twice using the compound of formula 1-1 obtained in above step 4, as recovered above, and quinidine, and then the process of (a) was finally performed.

(d) The compound of formula 2a-1 (1 eq, 135 g) obtained from the reactor and EtOH (20 vol, 2700 ml) were added thereto, and the inside of the reactor was warmed to 70 to 80°C. The inside of the reactor was stirred for one to two hours, cooled to 20 to 30°C, stirred for one hour, filtered (washed with EtOH (3 vol, 405 ml)), and dried under vacuum (yield 74.99%, ee 99.32%).

¹H NMR (500 MHz, DMSO-d₆)δppm 1.12(m, 1H), 1.72(m, 2H), 1.87(m, 1H), 2.22(m, 1H), 2.30(m, 1H), 2.52-2.64(m, 2H), 3.08(m, 1H), 3.15(s, 3H), 3.17(s, 1H), 3.32(m, 2H), 3.47(m, 1H), 3.86(m, 1H), 4.00(m, 3H), 4.24-4.30(m, 1H), 4.40-4.49(m, 1H), 4.77(br d, *J*=4.58Hz, 1H), 5.15-5.25(m, 2H), 6.04-6-18(m, 2H), 6.44(br s, 1H), 7.38-7.44(m, 2H), 7.58-7.65(m, 2H), 7.70(s, 1H), 7.94(s, 1H), 7.96(d, *J*=9.16Hz, 1H), 8.75(m, 1H)

### (Step 6) Synthesis of (S)-4-(methylsulfonyl)-3,4-dihydro-2H-thieno[3,2-g]chromen-7-carboxylic acid (Formula 1a-1)

The compound of formula 2a-1 (1.0 eq, 120 g) obtained in above step 5 and MeOH (5 vol, 600 ml) were added to a reactor, and 2 N HCl (50 vol, 6000 ml) was added dropwise thereto. The reaction solution was stirred for one hour while maintaining 20 to 30°C, and IPC was confirmed. When quinidine is < 1.0%, the reaction was completed. The resulting solid was filtered and sequentially washed with water (5 vol, 600 ml), cold EtOH (1 vol, 120 ml), and heptane (3 vol, 360 ml). The filtered solid was dried under vacuum to obtain the title compound (ee 99.50%, purity 99.86%, yield 91.6%, total yield 68.69% in steps 5 and 6).

¹H NMR (500 MHz, DMSO-d₆)δppm 2.28-2.37(m, 1H), 2.54-2.70(m, 1H), 3.16(s, 3H), 4.29-4.35(m, 1H), 4.48(td, *J*=11.48, 2.44Hz, 1H), 4.81(br d, *J*=4.40Hz, 1H), 7.54(s, 1H), 8.06(s, 2H), 13.33(br s, 1H)

### (Step 7) Synthesis of (S)-N-(2-chloro-6-(4-chlorophenoxy)pyridin-4-yl)-4-(methylsulfonyl)-3,4-dihydro-2H-thieno[3,2-g]chromen-7-carboxamide (Formula I-1)

The compound of formula 1a-1 (1 eq, 45 g) obtained in above step 6, the compound of formula 3-1 (1.2 eq, 44.1 g) obtained in above preparation example, pyridine (10 vol, 450 ml), and water (0.06 vol, 27 ml) were added to a reactor, and the resulting mixture was cooled to 0±5°C. POCl₃ (0.5 eq, 11.0 g) was added in three portions, and then the inside of the reactor was warmed to 10±5°C and stirred for one hour. When the compound of formula 1a-1 obtained in above step 6 was < 1.0%, the reaction was completed, and 2 N HCl (20 vol, 900 ml) was slowly added dropwise thereto at the same temperature for two hours. The reaction solution was warmed to 20 to 30°C, stirred for one hour, and filtered (washed with water (10 vol, 450 ml)). A filtered solid was added to the reactor, and water (10 vol, 450 ml) and 2 N HCl (10 vol, 450 ml) were added thereto, stirred for one hour, filtered (washed with water (10 vol, 450 ml)), and dried under vacuum to obtain the title compound (yield 103.98%, purity 96.34%).

¹H NMR (500 MHz, DMSO-d₆)δppm 2.30-2.38(m, 1H), 2.59-2.66(m, 1H), 3.16(s, 3H), 4.30-4.34(m, 1H), 4.45-4.52(m, 1H), 4.81(br d, *J*=3.91, 1H), 7.26(m, 2H), 7.31(s, 1H), 7.52(m, 2H), 7.58(s, 1H), 7.66(s, 1H), 8.06(s, 1H), 8.31(s, 1H), 10.93(s, 1H)

### (Step 8) Synthesis of THF solvate of (S)-N-(2-chloro-6-(4-chlorophenoxy)pyridin-4-yl)-4-(methylsulfonyl)-3,4-dihydro-2H-thieno[3,2-g]chromen-7-carboxamide

The compound of formula I-1 (1.0 eq, 80 g) obtained in above step 7, THF (9 vol, 720 ml) and water (0.5 vol, 40 ml) were added to a reactor, and dissolved therein. The dissolved solution was filtered using a 0.65 µm housing filter and washed with THF (1 vol, 80 ml). Water (20 vol, 1600 ml) was added to the reactor containing the filtered solution, stirred at 20 to 30°C for one hour, and then filtered (30% THF in H₂O (2 vol, 160 ml) as a washing solution). The filtered solid was dried under vacuum to confirm L-form (XRD: L-form confirmed). A dried solid was added to the reactor, and THF (5 vol, 400 ml) was added thereto, warmed to 60 to 70°C, and stirred for 0.5 to 1 hours. After cooling the inside of the reactor to 20 to 30°C, the resulting mixture was stirred at the same temperature for three hours and filtered. The resulting product was washed with cooled THF (2 vol, 160 ml) and dried under vacuum to obtain the title compound (yield 80.06%, purity 99.93%).

¹H NMR (500 MHz, DMSO-d₆) δppm 1.72-1.79(THF), 2.30-2.38(m, 1H), 2.62(br dd, *J*=15.14, 2.44Hz, 1H), 3.16(s, 3H), 3.57-3.62(THF), 4.30-4.35(m, 1H), 4.48(td, *J*=11.60, 2.69Hz, 1H), 4.81(br d, *J*=3.91Hz, 1H), 7.24-7.28(m, 2H), 7.31(s, 1H), 7.50-7.54(m, 2H), 7.58(s, 1H), 7.66(s, 1H), 8.07(s, 1H), 8.30(s, 1H), 10.92(s, 1H)

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A method for preparing a compound represented by formula I or a solvate thereof, the method comprising:
a step of preparing a compound represented by formula 2 using a compound represented by formula 1;
a step of preparing a compound represented by formula 1a using the compound represented by formula 2; and
a step of preparing a compound represented by formula I or a solvate thereof using the compound represented by formula 1a and a compound represented by formula 3: 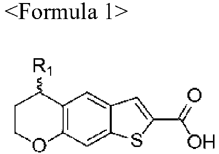 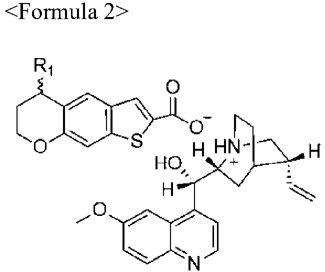 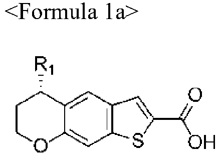 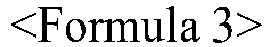 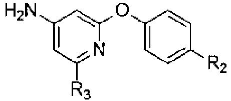 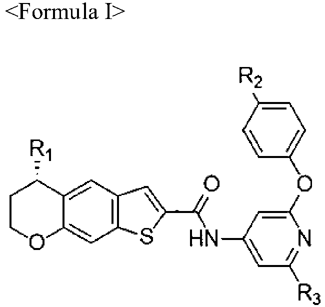
wherein in formulas 1, 2, 1a, 3 and I,
R₁ is -S(=O)(=Ra)Rb,
Ra is O or NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
at least one H of Ra or Rb is each independently substituted with F, Br, Cl or I, and
R₂ and R₃ are each independently F, Br, Cl or I.

2. The method of claim 1, wherein the step of preparing of the compound represented by formula 2 using the compound represented by formula 1 comprises a step of reacting the compound represented by formula 1 with quinidine.

3. The method of claim 1, wherein the step of preparing of the compound represented by formula 2 using the compound represented by formula 1 comprises a step of reacting the compound represented by formula 1 with quinidine in a presence of methanol, ethanol, propanol, isopropanol, butanol, or a mixture thereof.

4. The method of claim 1, wherein the step of preparing of the compound represented by formula 2 using the compound represented by formula 1 comprises a step of reacting the compound represented by formula 1 with quinidine at 0 to 90°C.

5. The method of claim 2, wherein the compound represented by formula 1 and quinidine are mixed at an equivalent ratio of 1:0.8 to 1:1.3.

6. The method of claim 3, wherein methanol is mixed in a volume of 1 to 40 times a weight of the compound represented by formula 1.

7. The method of claim 3, wherein ethanol is mixed in a volume of 3 to 60 times a weight of the compound represented by formula 1.

8. The method of claim 1, wherein a step of recovering quinidine and a step of recovering the compound represented by formula 1 are further comprised between the step of preparing of the compound represented by formula 2 using the compound represented by formula 1 and the step of preparing of the compound represented by formula 1a using the compound represented by formula 2.

9. The method of claim 8, wherein the step of preparing of the compound represented by formula 2 using the compound represented by formula 1, the step of recovering of quinidine, and the step of recovering of the compound represented by formula 1 are repeated.

10. The method of claim 9, wherein the repeating is performed twice or more and ten times or less.

11. The method of claim 8, wherein the step of recovering of quinidine is performed by adding 1,8-diazabicyclo[5.4.0]undec-7-ene.

12. The method of claim 8, wherein the step of recovering of the compound represented by formula 1 is performed by adding hydrochloric acid, sulfuric acid, acetic acid, or trifluoroacetic acid.

13. The method of claim 1, wherein the step of preparing of the compound represented by formula 1a using the compound represented by formula 2 is performed by adding hydrochloric acid, sulfuric acid, acetic acid, or trifluoroacetic acid.

14. The method of claim 1, wherein the step of preparing of the compound represented by formula I or the solvate thereof using the compound represented by formula 1a and the compound represented by formula 3 is performed through an amidation reaction.

15. The method of claim 1, wherein
the compound represented by formula 1 is a compound represented by formula 1-1,
the compound represented by formula 2 is a compound represented by formula 2-1,
the compound represented by formula 1a is a compound represented by formula 1a-1,
the compound represented by formula 3 is a compound represented by formula 3-1, and
the compound represented by formula I is a compound represented by formula I-1:

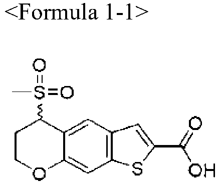

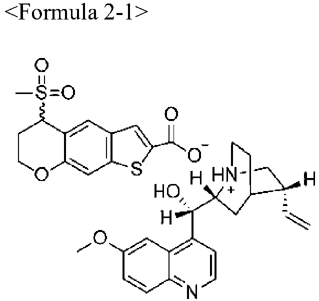

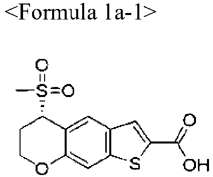

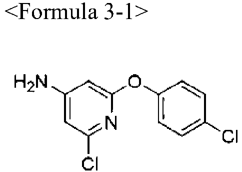

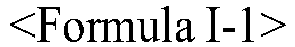

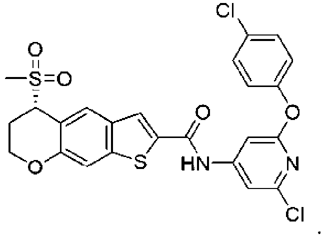

16. The method of claim 15, wherein the compound represented by formula 1-1 is prepared by a method comprising:
a step of preparing a compound represented by formula 6 using a compound represented by formula 4 and a compound represented by formula 5;
a step of preparing a compound represented by formula 7 using the compound represented by formula 6;
a step of preparing a compound represented by formula 8 using the compound represented by formula 7; and
a step of preparing a compound represented by formula 1-1 using the compound represented by formula 8:

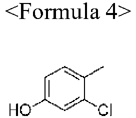

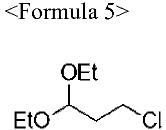

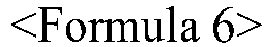

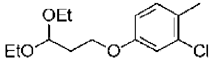

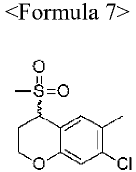

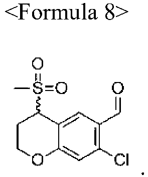

17. An intermediate compound represented by formula 2 below for preparing a compound represented by formula I or a solvate thereof: 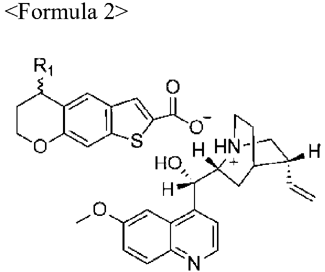 wherein in formula 2,
R₁ is -S(=O)(=Ra)Rb,
Ra is O or NH,
Rb is H, C1-6 alkyl, C1-6 alkoxy-C 1-6 alkyl, C1-6 alkylcarbonyl-C1-6 alkyl, C2-7 alkenyl, amino, or amino C1-6 alkyl, and
at least one H of Ra or Rb is each independently substituted with F, Br, Cl or I.

18. The intermediate compound of claim 17, wherein the compound represented by formula 2 is a compound represented by formula 2-1 below:

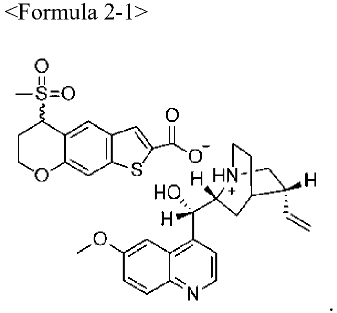

19. A method for preparing an intermediate compound according to claim 17, the method comprising:
a step of preparing a compound represented by formula 2 using a compound represented by formula 1: 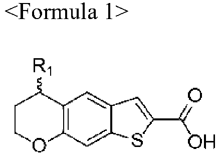 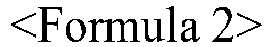 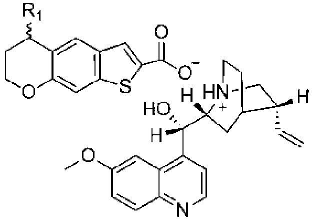
wherein in formulas 1 and 2,
R₁ is -S(=O)(=Ra)Rb,
Ra is O or NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl, and
at least one H of Ra or Rb is each independently substituted with F, Br, Cl or I.
